# EUROPEAN PATENT APPLICATION

(11) **EP 4 427 668 A1**
(43) Date of publication of application: **11.09.2024**
(21) Application number: 24162486.5
(22) Date of filing: 08.03.2024
(51) Int. Cl.: A61B 5/02, A61B 5/026, A61B 5/107, A61B 5/1455, A61B 5/00, A61B 3/14, A61B 5/18

(54) **OCULAR DERIVED BLOOD FLOW AND OXYGENATION VIA EYE TRACKING**

(30) Priority: 10.03.2023 US 202363451280 P; 05.03.2024 US 202418595628
(71) Applicant: Rockwell Collins, Inc., Cedar Rapids, IA 52498 (US)
(72) Inventor: SHAPIRO, Geoffrey A., Cedar Rapids, 52402 (US)
(74) Representative: Dehns

(57) **Abstract**

A system and method for monitoring blood oxygen levels via an eye tracking camera (104, 200) includes a computer system with a processor (100) configured to identify capillaries in the image stream of a pilot's eye. Changes in capillary size over time is directly correlated to blood flow levels. The processor (100) may monitor capillary color. Changes in capillary color is directly correlated to blood oxygen levels. The processor (100) may take remedial action when the pilot's blood oxygen level drops below a threshold. Such remedial action may include alerting the pilot, automatically alerting ground control crew, applying increasing levels of flight automation, etc.

## Description

### PRIORITY

The present application claims the benefit under 35 U.S.C. § 119(e) of U.S. Provisional App. No. 63/451,280 (filed March 10, 2023).

### BACKGROUND

Hypoxic hypoxia is a leading safety issue for pilots, including the United States Airforce, which has led to loss of aircraft and life. Existing methodologies for monitoring blood oxygen saturation and blood perfusion rely on skin contact, creating many problems in the field including comfort issues and sensor disruption due to the operational environment. It would be advantageous to have non-invasive methods to measure and mitigate low blood oxygen levels.

### SUMMARY

In one aspect, embodiments of the inventive concepts disclosed herein are directed to a system and method for monitoring blood oxygen levels via an eye tracking camera. A computer system includes an eye tracking camera; a processor is configured to identify capillaries in the image stream of a pilot's eye. Changes in capillary size over time is directly correlated to blood flow levels.

In a further aspect, the processor may monitor capillary color. Changes in capillary color is directly correlated to blood oxygen levels.

In a further aspect, the processor may take remedial action when the pilot's blood oxygen level drops below a threshold. Such remedial action may include alerting the pilot, automatically alerting ground control crew, applying increasing levels of flight automation, etc.

It is to be understood that both the foregoing general description and the following detailed description are exemplary and explanatory only and should not restrict the scope of the claims. The accompanying drawings, which are incorporated in and constitute a part of the specification, illustrate exemplary embodiments of the inventive concepts disclosed herein and together with the general description, serve to explain the principles.

### BRIEF DESCRIPTION OF THE DRAWINGS

The numerous advantages of the embodiments of the inventive concepts disclosed herein may be better understood by those skilled in the art by reference to the accompanying figures in which:
- FIG. 1: shows a block diagram of a system for implementing exemplary embodiments;
- FIG. 2: shows an environmental view of a helmet mounted device useful for implementing exemplary embodiments; and
- FIG. 3: shows a block diagram of a neural network useful for implementing exemplary embodiments.

### DETAILED DESCRIPTION

Before explaining various embodiments of the inventive concepts disclosed herein in detail, it is to be understood that the inventive concepts are not limited in their application to the arrangement of the components or steps or methodologies set forth in the following description or illustrated in the drawings. In the following detailed description of embodiments of the instant inventive concepts, numerous specific details are set forth in order to provide a more thorough understanding of the inventive concepts. However, it will be apparent to one of ordinary skill in the art having the benefit of the instant disclosure that the inventive concepts disclosed herein may be practiced without these specific details. In other instances, well-known features may not be described in detail to avoid unnecessarily complicating the instant disclosure. The inventive concepts disclosed herein are capable of other embodiments or of being practiced or carried out in various ways. Also, it is to be understood that the phraseology and terminology employed herein is for the purpose of description and should not be regarded as limiting.

**As** used herein a letter following a reference numeral is intended to reference an embodiment of a feature or element that may be similar, but not necessarily identical, to a previously described element or feature bearing the same reference numeral (e.g., 1, 1a, 1b). Such shorthand notations are used for purposes of convenience only, and should not be construed to limit the inventive concepts disclosed herein in any way unless expressly stated to the contrary.

Further, unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of "a" or "an" are employed to describe elements and components of embodiments of the instant inventive concepts. This is done merely for convenience and to give a general sense of the inventive concepts, and "a" and "an" are intended to include one or at least one and the singular also includes the plural unless it is obvious that it is meant otherwise.

Also, while various components may be depicted as being connected directly, direct connection is not a requirement. Components may be in data communication with intervening components that are not illustrated or described.

Finally, as used herein any reference to "one embodiment," or "some embodiments" means that a particular element, feature, structure, or characteristic described in connection with the embodiment is included in at least one embodiment of the inventive concepts disclosed herein. The appearances of the phrase "in at least one embodiment" in the specification does not necessarily refer to the same embodiment. Embodiments of the inventive concepts disclosed may include one or more of the features expressly described or inherently present herein, or any combination or sub-combination of two or more such features.

Broadly, embodiments of the inventive concepts disclosed herein are directed to a system and method for monitoring blood oxygen levels via an eye tracking camera. A computer system includes an eye tracking camera; a processor is configured to identify capillaries in the image stream of a pilot's eye. Changes in capillary size over time is directly correlated to blood flow levels. The processor may monitor capillary color. Changes in capillary color is directly correlated to blood oxygen levels. The processor may take remedial action when the pilot's blood oxygen level drops below a threshold. Such remedial action may include alerting the pilot, automatically alerting ground control crew, applying increasing levels of flight automation, etc.

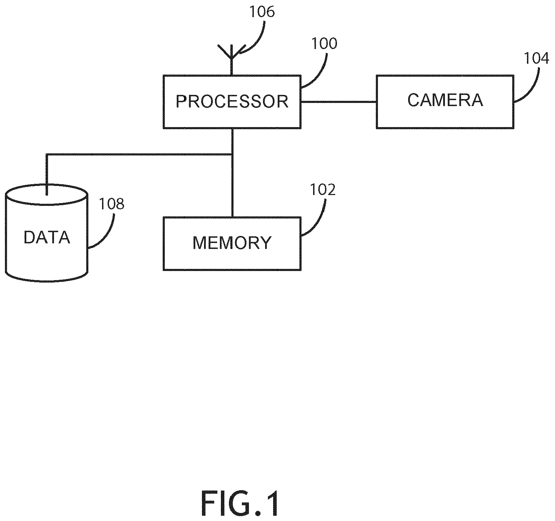

Referring to FIG. 1, a block diagram of a system for implementing exemplary embodiments is shown. The system includes a processor 100, memory 102 connected to the processor 100 for storing processor executable code, and at least one eye tracking camera 104. The processor 100 receives an image stream from the eye tracking camera 104 and continuously identifies capillaries in a user's eye.

In at least one embodiment, the processor 100 measures the width and chromaticity of the ocular capillaries. The width is correlated to the amount of blood flowing through the eye which in turn is associated with cranial blood flow. Blood flow may very across different parts of a user's body, and cranial blood flow is a key indicator of hypoxia. Alternatively, or in addition, a chromaticity shift of the capillaries may indicate a change in blood oxygen levels. Blood appears more towards the blue spectrum as blood oxygen levels drop; by identifying chromaticity changes over time, the processor 100 may infer blood oxygen saturation. Chromaticity may be determined by applying various filters to the image stream.

In at least one embodiment, the processor 100 may be configured according to an artificial intelligence / machine learning algorithm to produce blood flow and blood oxygen determinations based on the image stream from the eye tracking camera 104.

In at lest one embodiment, the system may include a data storage element 108 in data communication with the processor 100. The processor 100 may maintain and retrieve a user specific ocular profile from the data storage element 108, and compare the image stream to the user specific profile. Such user specific profile may define a range of capillary width and chromaticity for the user for a more specific determination of blood oxygen levels.

In at least one embodiment, the processor 100 is configured to determine if the blood oxygen level of the user falls below one or more predetermined thresholds. When the processor 100 determines that the user's blood oxygen level has fallen below a threshold, the processor 100 may take some remedial action. For example, the processor 100 may alert the user or alert a remote monitoring personnel via a data communication element 106. Alternatively, or in addition, the processor 100 may instruct separate system to engage some increased level of automation. For example, when embodied in a pilot's helmet, the processor 100 may instruct an avionics system to assume some level of control of the aircraft.

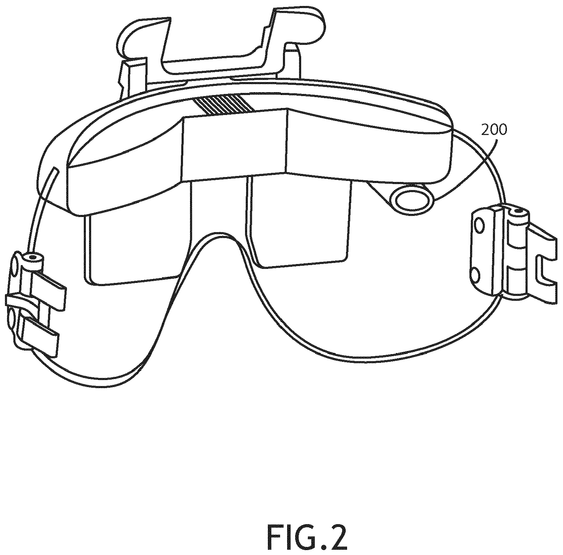

Referring to FIG. 2, an environmental view of a helmet mounted device useful for implementing exemplary embodiments is shown. The helmet mounted device includes an eye tracking camera 200 disposed for continuous, unobstructed view of the pilot's eye. Such eye tracking camera 200 may be disposed as not to obstruct the pilot's view while also providing an image stream of the sclera of the pilot's eye. Alternatively, or in addition, the eye tracking camera 200 may be disposed for a continuous view of both external capillaries and internal capillaries as visible through the pilot's iris.

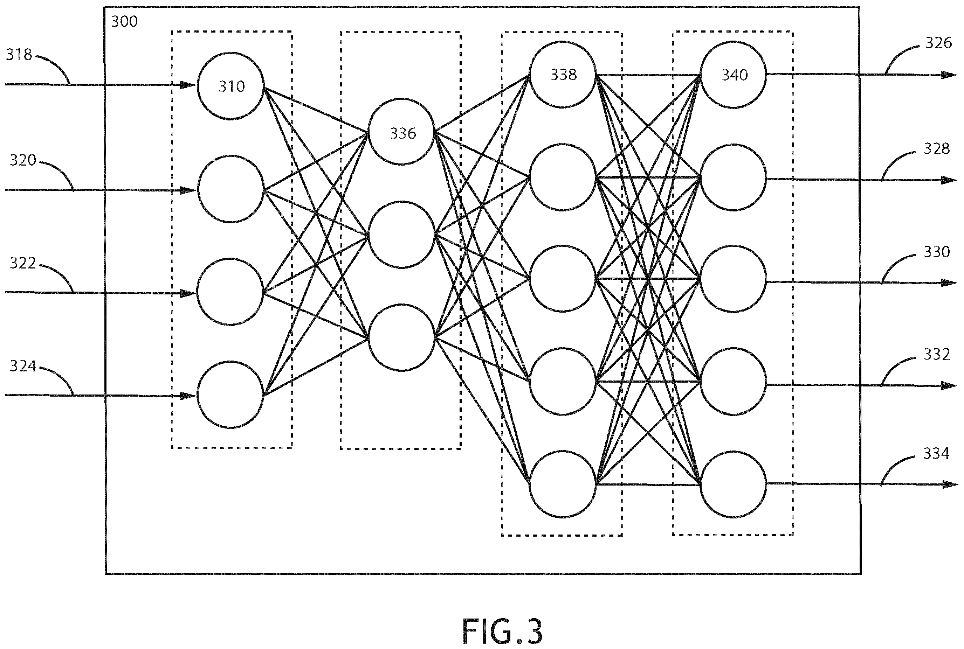

Referring to FIG. 3, a block diagram of a neural network 300 useful for implementing exemplary embodiments is shown. The neural network 300 comprises an input layer 302, and output layer 304, and a plurality of internal layers 306, 308. The input layer 302 may receive components of a streaming image from an eye tracking camera. Each layer comprises a plurality of neurons or nodes 310, 336, 338, 340. In the input layer 302, each node 310 receives one or more inputs 318, 320, 322, 324 corresponding to a digital signal and produces an output 312 based on an activation function unique to each node 310 in the input layer 302. An activation function may be a Hyperbolic tangent function, a linear output function, and / or a logistic function, or some combination thereof, and different nodes 310, 336, 338, 340 may utilize different types of activation functions. In at least one embodiment, such activation function comprises the sum of each input multiplied by a synaptic weight. The output 312 may comprise a real value with a defined range or a Boolean value if the activation function surpasses a defined threshold. Such ranges and thresholds may be defined during a training process. Furthermore, the synaptic weights are determined during the training process.

An output layer 304 including one or more output nodes 340 receives the outputs 316 from each of the nodes 338 in the previous intermediate layer 308. Each output node 340 produces a final output 326, 328, 330, 332, 334 via processing the previous layer inputs 316. Such outputs may comprise separate components of an interleaved input signal, bits for delivery to a register, or other digital output based on a n input signal and DSP algorithm. Final outputs 326, 328, 330, 332, 334 may comprise a characterization of ocular capillary size and chromaticity, a blood flow and blood oxygen estimation, a suggested remediation action based on blood flow and blood oxygen estimation, or the like.

Embodiments of the present disclosure enable sensor methodologies for hypoxia mitigation. Such embodiments are superior to existing methods because they do not rely on contact or a sensor in the field of view of the pilot.

It is believed that the inventive concepts disclosed herein and many of their attendant advantages will be understood by the foregoing description of embodiments of the inventive concepts, and it will be apparent that various changes may be made in the form, construction, and arrangement of the components thereof without departing from the broad scope of the inventive concepts disclosed herein or without sacrificing all of their material advantages; and individual features from various embodiments may be combined to arrive at other embodiments. The forms herein before described being merely explanatory embodiments thereof, it is the intention of the following claims to encompass and include such changes. Furthermore, any of the features disclosed in relation to any of the individual embodiments may be incorporated into any other embodiment.

## Claims

1. A computer apparatus comprising:
an eye tracking camera (104, 200); and
at least one processor (100) in data communication with the eye tracking camera (104, 200) and a memory storing processor executable code for configuring the at least one processor (100) to:
identify capillaries in an eye from an image stream from the eye tracking camera (104, 200);
continuously characterize a width of the identified capillaries; and
determine a cranial blood flow level a pilot based on the width characterization.

2. The computer apparatus of Claim 1, wherein the at least one processor (100) is further configured to identify hypoxia based on a threshold cranial blood flow level.

3. The computer apparatus of any preceding Claim, wherein the at least one processor (100) is further configured to:
continuously characterize a chromaticity of the identified capillaries; and
determine a blood oxygen saturation of the pilot based on the chromaticity characterization, and/or.
wherein the at least one processor (100) is further configured to apply one or more filters to the image stream to characterize the chromaticity.

4. The computer apparatus of any preceding Claim, wherein the at least one processor (100) is further configured to:
determine that at least one of the cranial blood flow level and the blood oxygen saturation has fallen below a threshold; and
execute a remedial action.

5. The computer apparatus of any preceding Claim, wherein:
the at least one processor (100) is further configured to retrieve a user specific ocular profile defining a range of capillary widths and chromaticity for the pilot; and
determining the cranial blood flow level and determining the blood oxygen saturation comprises comparing the characterized width and characterized chromaticity to the user specific ocular profile.

6. The computer apparatus of any preceding Claim, wherein the processor (100) embodies a trained neural network.

7. A method of monitoring a user's blood oxygenation comprising:
identifying capillaries in an eye from an image stream;
continuously characterizing a width of the identified capillaries;
determining a cranial blood flow level a pilot based on the width characterization;
continuously characterizing a chromaticity of the identified capillaries; and
determine a blood oxygen saturation of the user based on the chromaticity characterization.

8. The method of Claim 7, further comprising identifying hypoxia based on a threshold cranial blood flow level, and/or further comprising applying one or more filters to the image stream to characterize the chromaticity.

9. The method of Claim 7 or 8, further comprising:
determining that at least one of the cranial blood flow level and the blood oxygen saturation has fallen below a threshold; and
executing a remedial action.

10. The method of any of Claims 7 to 9, further comprising retrieving a user specific ocular profile defining a range of capillary widths and chromaticity for the pilot, wherein determining the cranial blood flow level and determining the blood oxygen saturation comprises comparing the characterized width and characterized chromaticity to the user specific ocular profile.

11. A pilot monitoring system comprising:
a helmet mounted device including an eye tracking camera (104, 200); and
at least one processor (100) in data communication with the eye tracking camera (104, 200) and a memory storing processor executable code for configuring the at least one processor (100) to:
identify capillaries in an eye from an image stream from the eye tracking camera (104, 200);
continuously characterize a chromaticity of the identified capillaries; and
determine a blood oxygen saturation of the pilot based on the chromaticity characterization.

12. The pilot monitoring system of Claim 11, wherein the at least one processor (100) is further configured to:
continuously characterize a width of the identified capillaries; and
determine a cranial blood flow level the pilot based on the width characterization.

13. The pilot monitoring system of Claim 11 or 12, wherein the at least one processor (100) is further configured to identify hypoxia based on a threshold cranial blood flow level, and/or . wherein the at least one processor (100) is further configured to apply one or more filters to the image stream to characterize the chromaticity.

14. The pilot monitoring system of any of Claims 11 to 13, wherein the at least one processor (100) is further configured to:
determine that at least one of the cranial blood flow level and the blood oxygen saturation has fallen below a threshold; and
execute a remedial action, and optionally wherein the remedial action comprised instructing an avionics system to increase a level of automation.

15. The pilot monitoring system of any of Claims 11 to 14, wherein:
the at least one processor (100) is further configured to retrieve a user specific ocular profile defining a range of capillary widths and chromaticity for the pilot; and
determining the cranial blood flow level and determining the blood oxygen saturation comprises comparing the characterized width and characterized chromaticity to the user specific ocular profile, and/or wherein the processor (100) embodies a trained neural network.
